Europäisches Patentamt

European Patent Office

Office européen des brevets

(11) Publication number: **0 240 188**
**A2**

## (12) EUROPEAN PATENT APPLICATION

(21) Application number: **87302060.6**

(22) Date of filing: **10.03.87**

(51) Int. Cl.⁴: **F25J 3/00** , C07C 9/04

(30) Priority: **01.04.86 US 847065**

(43) Date of publication of application:
**07.10.87 Bulletin 87/41**

(84) Designated Contracting States:
**BE DE ES FR GB GR IT NL SE**

(71) Applicant: **MCDERMOTT INTERNATIONAL, INC.**
**1010 Common Street PO Box 60035**
**New Orleans Louisiana 70160(US)**

(72) Inventor: **Aghili, Hafez Kermani**
**402 Houghton Road**
**Katy Texas 77450(US)**

(74) Representative: **Purvis, William Michael Cameron et al**
**D. Young & Co. 10 Staple Inn**
**London WC1V 7RD(GB)**

(54) Process for separating hydrocarbon gas constituents.

(57) A process for separating the gas components contained in liquified natural gas wherein a small amount of heavier reflux liquid (propane, butane, pentane, iso-butane and iso-pentane) is recycled to the top of a demethaniser column. This alters its chemical equilibrium causing methane to vapourise in large quantities and to separate from the liquified feed stream. A fractionator (70) receives the heavier demethanised liquid end product from the de-methaniser column (32) and through distillation sepa-rates the lighter ethanes and propanes from the heavier reflux liquid, a portion of the latter being recycled to the demethaniser column (32).

FIG.1

EP 0 240 188 A2

## PROCESS FOR SEPARATING HYDROCARBON GAS CONSTITUENTS

The invention relates to a method of separating hydrocarbon gas constituents such as methane, ethane and propane in natural gas.

The history of separating gas constituents from natural gas is a relatively recent one. In the 1940's the first prototype natural gas cryogenic turboexpander was built and in the 50's this turboexpander concept was applied to air plants, hydrogen plants, and helium purification plants. However, it was not until the 1960's that the first commercial natural gas turboexpander plant started operation. As commercial demand for these separated gases increased, many other such plants came into existence each with better and improved designs for separating the lighter elements (methane, ethane and propane) from the heavier elements (butane, pentane and their iso-components) contained in natural gas.

Many patents exist pertaining to these improvements with some of the more relevant patents being those to Gulsby (US-A-4,464,190 and US-A-4,453,958), Horton (US-A-4,410,342 and US-A-3,398,543), and Campbell, et al (US-A-4,278,457 and US-A-4,171,964). While each of these patents are improvements over their predecessors, none of them address the commercial need for nearly 100% gas separation and recovery.

According to the invention there is provided a process for separating the constituents of a gas stream characterised by the steps of:

(a) lowering the temperature of the gas stream;

(b) supplying the lower temperature gas stream to a high pressure separator, the high pressure separator separating the gas stream into a predominantly vapour stream and a predominantly fluid stream;

(c) lowering the pressure of the predominantly vapour stream;

(d) supplying the lower pressure vapour stream to an upper region of a demethaniser column;

(e) lowering the pressure of the predominantly fluid stream;

(f) supplying the lower pressure fluid stream to the demethaniser column at an elevation below the level at which the vapour stream is supplied to the column;

(g) removing cold vapour residue gas from an upper region of the demethaniser column, the vapour residue gas comprising predominantly methane and other residual light vapours;

(h) passing the vapour residue gas through at least one heat exchanger to raise the temperature of the vapour residue gas;

(i) compressing the vapour residue gas for delivery elsewhere;

(j) removing a cold demethanised product from a lower region of the demethaniser column;

(k) supplying at least a portion of the cold demethanised product to a fractionator, the fractionator operating as a distillation column;

(l) separating the cold demethanised product into an ethane overhead product and a deethanised bottom product;

(m) removing a generally liquid deethanised product from a lower region of the fractionator;

(n) drawing off a portion of the deethanised product;

(o) lowering the temperature of the drawn-off product; and

(p) supplying the lower temperature deethanised product to the top of the demethaniser column whereby the addition of the product alters the chemical equilibrium existing in the top of the demethaniser column thereby enhancing the recovery of ethane constituents at the expense of a small reduction in recovery of propane and heavier constituents.

The process of the invention can achieve controlled separation of the natural gas feed stream constituents thereby to affect the composition of product streams based on the economics of natural gas constituents. The process can utilise the distillation column and recycled streams from other parts of the plant to affect the chemical equilibrium in the top section of the column for most economical product recovery. A return condensate line feeding the demethaniser column can favourably alter the chemical equilibrium in this demethaniser column.

The demethanising column in conjunction with reboilers, compressors and turboexpanders can separate the heavier liquid elements in the natural gas feed stream from the lighter vapour methane. This heavier liquid end product leaves the bottom of the demethaniser column where it enters the fractionator forming a distillation column. In the fractionator the lighter ethane and propane vapours are separated from the heavier liquid butanes, pentanes and their iso-components. A small portion of the heavier liquid end product from the fractionator, nearly all butane, pentane, iso-butane and iso-pentane, is recycled as reflux to the top of the demethaniser column. This additional amount of the heavier liquids in the demethaniser column alters the chemical equilibrium existing in this upper region of the demethaniser where the elements are predominantly all lighter vapourised gases. This reflux addition and the resulting change in equilibrium causes nearly 100% gas separation of the methane in the demethaniser thereby providing a pure demethanised end product while only sacrificing a small volume of the heavier liquids to achieve this result.

The invention is diagrammatically illustrated by way of example in the accompanying drawings, in which:-

Figure 1 is a schematic illustration of apparatus for carrying out a process according to the invention including a demethaniser, a fractionator and a return reflux line;

Figure 2 is a schematic illustration of apparatus for carrying out a process according to the invention wherein a demethanised recycled absorption stream from a fractionator is initially cooled by a chiller and then a heat exchanger;

Figure 3 is a schematic illustration of apparatus for carrying out a process according to the invention including a deethaniser fractionator and a depropaniser fractionator; and

Figure 4 is a schematic illustration similar to Figure 3 but wherein a recycled depropanised product stream is initially cooled by a chiller and then a heat exchanger.

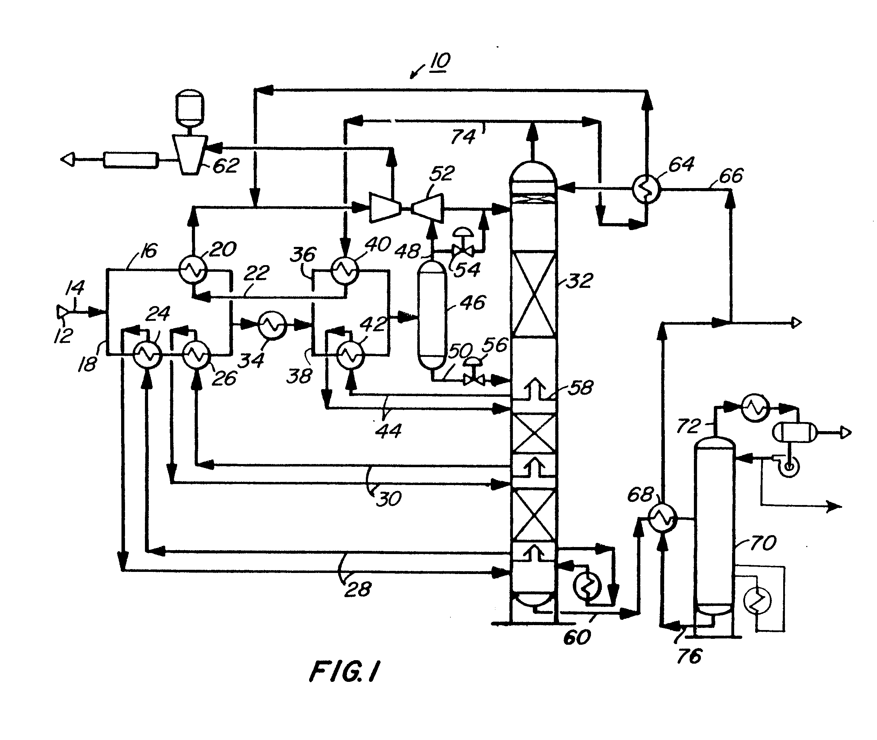

Referring to Figure 1, natural gas enters gas separator process apparatus 10 through an inlet 12 after having first been dehydrated. The gas passes to line 14 and is then divided into two separate streams 16 and 18 respectively. The gas stream 16 is cooled in a warm gas/gas exchanger 20 by cool residue gas in a line 22. The gas stream 18 is cooled in a reboiler 24 and a lower side heater 26 through which demethaniser liquid flows via lines 28 and 30 from a demethaniser column 32.

From the exchanger 20 and the lower side heater 26, the cooled gas streams 16 and 18 recombine and enter a gas chiller 34 where the combined stream is further cooled by a refrigerant. After the chiller 34, the chilled stream is again separated into two streams 36 and 38 for more cooling. The stream 36 is cooled in a cold gas/gas exchanger 40 by cold residue gas directly from the top of the demethaniser column 32. This residue gas is generally at a temperature of -95.5°C (-140°F). As shown, this cold residue gas passes first through the cold gas/gas exchanger 40 before travelling through the warm gas/gas exchanger 20 via the line 22. The stream 38 is cooled in an upper side heater 42 by demethanised liquid flowing through a line 44 from the demethaniser column 32. This demethaniser liquid is generally at a temperature of -69.4°C (-125°F). The cold gas streams 36 and 38 then recombine and enter a high pressure separator 46 where the cooled inlet gas is separated into a gas stream 48 and a liquid stream 50. The gas stream 48, which by this time consists predominantly of the lighter methanes, ethanes and propanes, is expanded to reduce its pressure such as by a main expander 52 or across an expansion valve 54. This expansion further cools the gas before it is fed into an upper region of the demethaniser column 32. The condensed liquid stream 50 from the high pressure separator 46 is also expanded, thereby reducing its pressure, such as across an expansion valve 56, before entering the side of the demethaniser column 32. By this time, the liquid stream 50 consists predominantly of the heavier butanes, pentanes and their iso-components.

As liquid is fed to the demethaniser column 32, it flows downward under the force of gravity. During its journey, the liquid is engaged by rising vapours which strip the methane from this liquid as it passes through trays 58 in the demethaniser column 32. This stripping operation produces a demethanised end product which is removed from the bottom of the demethaniser column 32 via a line 60. The rising methane vapours are generated from the heat obtained from the heat exchangers 24, 26, 42 via the lines 28, 30 and 44.

A portion of the residue from the top of the demethaniser column 32, where its temperature is approximately -95.5°C (-140°F), is conveyed to the cold gas/gas exchanger 40 and the warm, gas/gas exchanger 20. This cold residue cools the incoming gas streams 36 and 16. From these heat exchangers, the warmed residue is compressed by the compressor side of the main expander 52 and then by a turbo recompressor 62 after which it is cooled and transported elsewhere. The remaining portion of the cold residue from the top of the demethaniser column 32 passes through a heat exchanger 64 which chills the recycled absorption stream in a feed line 66 that is recycled to the top of the demethaniser column 32.

The line 60 which forms an exit at the bottom of the demethaniser column 32 transports the demethanised end product from the column 32 to a heat exchanger 68 which heats this product before it enters a fractionator 70. The fractionator 70 acts as a distillation column or deethaniser and separates the lighter ethane component in this end product from the heavier propane, butanes, pentanes and their iso-components. The overhead product from the fractionator 70 exits through a line 72, which line 72 contains essentially 100% of the ethane. A lower line 76, connected to the bottom of the fractionator 70, transports the deethanised product from the fractionator 70 through the exchanger 68 where this deethanised end product heats the incoming demethanised end product in the line 60. This deethaniser end product from the fractionator 70 is then recycled to the top of the demethaniser column 32 via the line 66. The addition of this portion of the heavier deethanised product to the demethaniser column 32 alters significantly the chemical equilibrium in the top of the column 32. This causes the lighter vapourised ethane in the top of the column 32 to become liquid and fall to the bottom of the column 32 while a small amount of propane and heavier constituents are vapourised to make up the dew point in a stream 74. The feed line 66 acts as an absorption medium which enhances the recovery of ethane at the expense of a small reduction in the recovery of propane and heavier components.

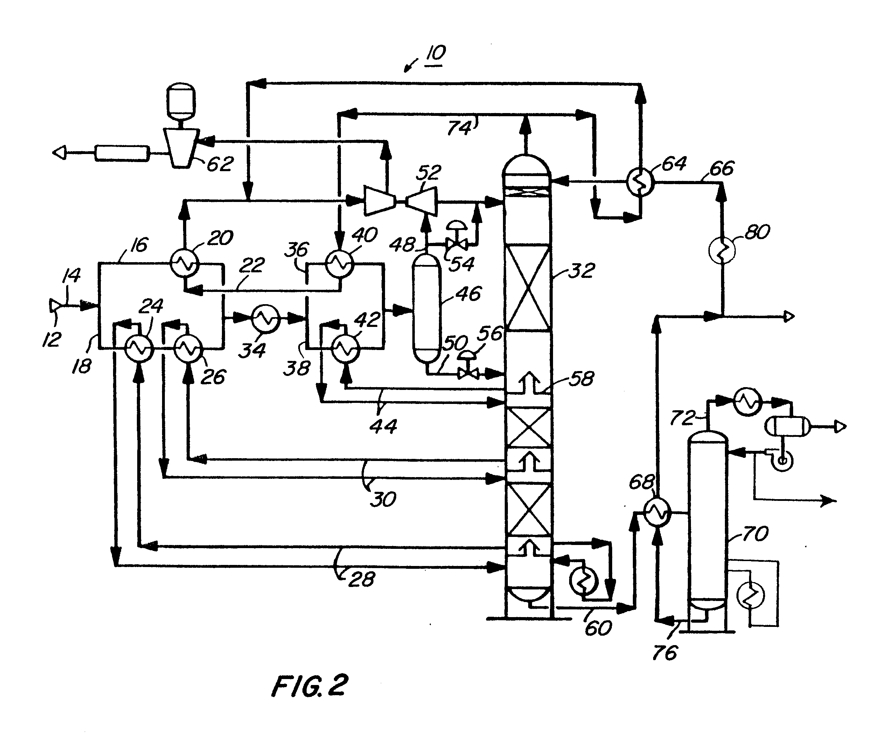

Another arrangement for the heat exchange of the recycled absorption in the feed line 66 is shown in Figure 2. In this case, the recycled absorption feed line 66 is first cooled by heat exchange in a chiller 80 and then is further cooled in the exchanger 64 by a portion of the residue gas from the demethaniser column 32.

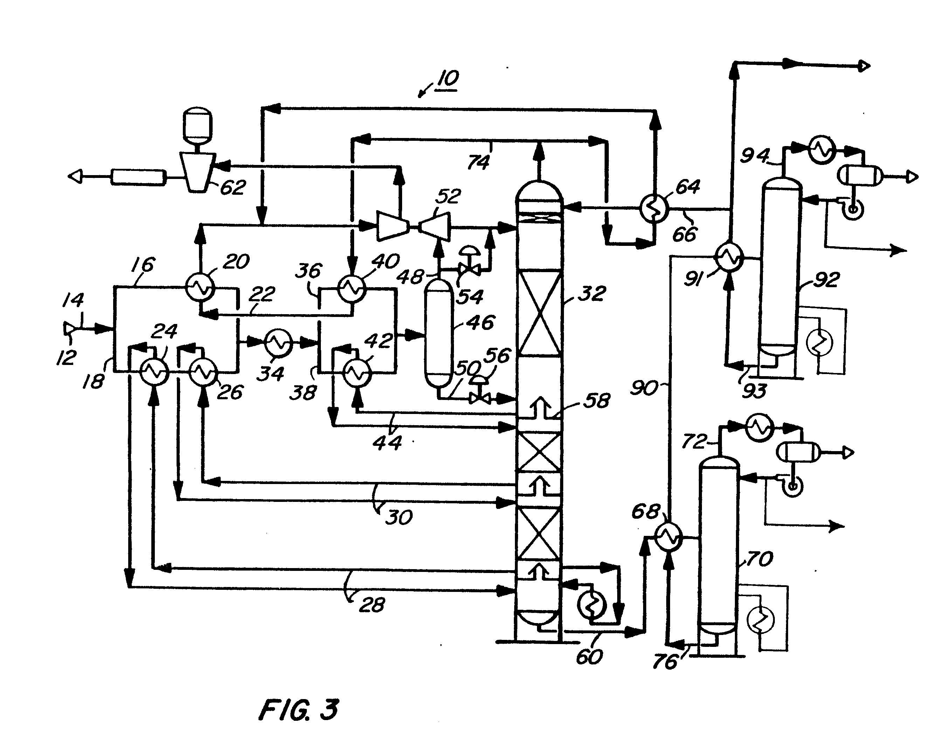

Figure 3 illustrates another variation of this process which uses a depropanised product stream as the absorption medium for the demethaniser column 32. In this case, the bottom deethanised product from the fractionator 70 flowing through a line 90 is heated in a heat exchanger 91 before entering a depropaniser or fractionator 92. The fractionator 92 separates this incoming deethanised product into a top product that is predominantly propane and which leaves the fractionator 92 via a line 94, and a depropanised bottom product that consists of butanes and the heavier constituents and which leaves the fractionator 92 via a line 93. A portion of this heavier depropanised product in the line 93 is diverted to the line 66 where it is cooled in the heat exchanger 64 by the residue gases from the demethaniser column 32 before entering the top of the demethaniser column 32. This action causes the lighter vapourised ethane and propane in the top of the column 32 to become liquid and fall to the bottom while a small amount of butane and heavier constituent are vapourised to make up the dew point in the stream 74.

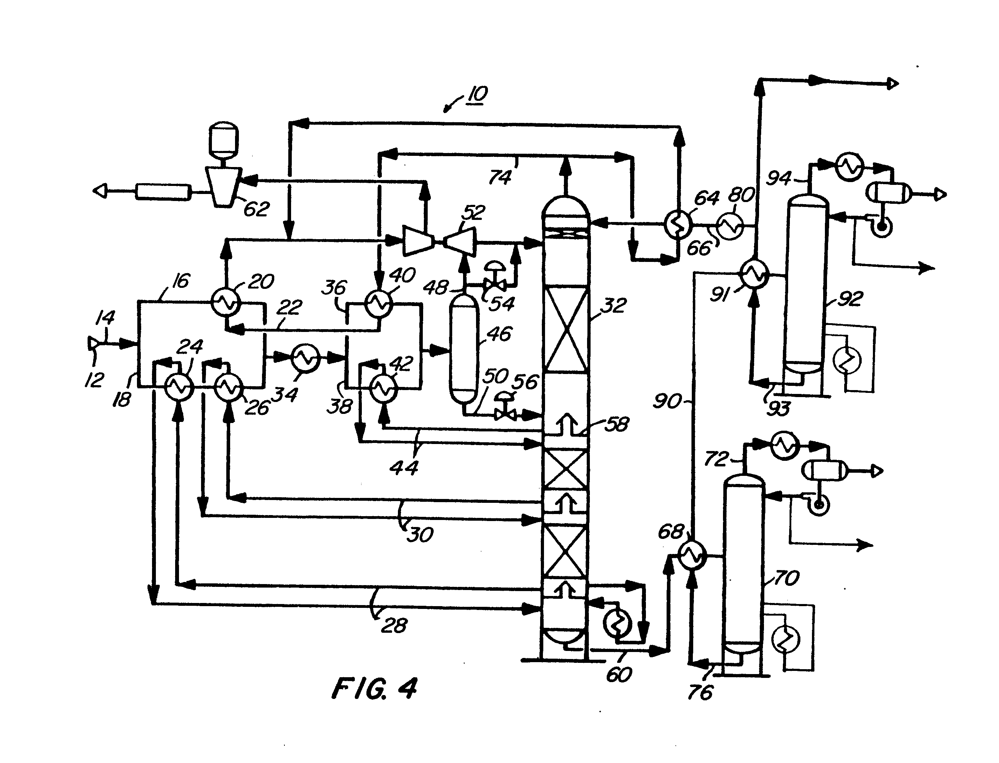

Another arrangement of the recycle absorption medium is shown in Figure 4. In this case, the recycle absorption stream 66 is first cooled by heat exchange in the chiller 80 with a refrigerant and then is further cooled in the heat exchanger 64 by heat exchange with a portion of the residue gas from the column 32.

## Claims

1. A process for separating the constituents of a gas stream characterised by the steps of:

(a) lowering the temperature of the gas stream;

(b) supplying the lower temperature gas stream to a high pressure separator (46), the high pressure separator (46) separating the gas stream into a predominantly vapour stream (48) and a predominantly fluid stream (50);

(c) lowering the pressure of the predominantly vapour stream (48);

(d) supplying the lower pressure vapour stream (48) to an upper region of a demethaniser column (32);

(e) lowering the pressure of the predominantly fluid stream (50);

(f) supplying the lower pressure fluid stream (50) to the demethaniser column (32) at an elevation below the level at which the vapour stream (48) is supplied to the column;

(g) removing cold vapour residue gas from an upper region of the demethaniser column (32), the vapour residue gas comprising predominantly methane and other residual light vapours;

(h) passing the vapour residue gas through at least one heat exchanger (64) to raise the temperature of the vapour residue gas;

(i) compressing the vapour residue gas for delivery elsewhere;

(j) removing a cold demethanised product from a lower region of the demethaniser column (32);

(k) supplying at least a portion of the cold demethanised product to a fractionator (70), the fractionator operating as a distillation column;

(l) separating the cold demethanised product into an ethane overhead product and a deethanised bottom product;

(m) removing a generally liquid deethanised product from a lower region of the fractionator (70);

(n) drawing off a portion of the deethanised product;

(o) lowering the temperature of the drawn-off product; and

(p) supplying the lower temperature deethanised product to the top of the demethaniser column (32) whereby the addition of the product alters the chemical equilibrium existing in the top of the demethaniser column thereby enhancing the recovery of ethane constituents at the expense of a small reduction in recovery of propane and heavier constituents.

2. A process according to claim 1, wherein the temperature of the gas stream is lowered by separating the stream into two streams (16, 18) and cooling the streams (16, 18) in gas heat exchangers (20, 24, 26).

3. A process according to claim 2, wherein at least one (20) of the gas heat exchangers use the cold vapour residue gas as a refrigerant and another of the gas heat exchangers uses the cold demethanised product as a refrigerant.

4. A process according to claim 2 or claim 3, wherein the pressure of the predominantly vapour stream (48) is lowered across expansion means (52, 54).

5. A process according to claim 4, wherein the expansion means comprise a main expander (52).

6. A process according to claim 4, wherein the expansion means comprise an in-line expansion valve (54).

7. A process according to claim 2 or claim 3, wherein the pressure of the predominantly fluid stream (50) is lowered across an in-line expansion valve (56).

8. A process according to claim 7, wherein the vapour residue gas is compressed on the compressor side of an expander (52).

9. A process according to claim 8, wherein the temperature of the removed demethanised product is increased in a heat exchanger (68) using the deethanised end product as a heat exchange medium.

10. A process according to claim 9, further comprising the step of supplying at least a portion of the deethanised bottom product to a second fractionator (92), which second fractionator (92) separates the deethanised product into a propane overhead product and a depropanised bottom product.

11. A process according to claim 10, wherein the depropanised bottom product from the second fractionator (92) is cross exchanged with the deethanised bottom product feed to the second fractionator (92).

12. A process according to claim 11, wherein a portion of the depropanised bottom product is cooled and supplied to the top of the demethaniser column (32) and the addition of the cooled depropanised bottom product enhances the recovery of ethane and propane constituents in the demethaniser column (32).

FIG.1

FIG. 2

0 240 188

FIG. 3

0 240 188

FIG. 4